(19) Europäisches Patentamt
European Patent Office
Office européen des brevets

(11) Veröffentlichungsnummer: **0 257 518 B1**

(12) **EUROPÄISCHE PATENTSCHRIFT**

(45) Veröffentlichungstag der Patentschrift: **02.01.92**

(51) Int. Cl.5: **C07D 215/48**, A01N 43/42, A01N 47/06, A01N 47/18, A01N 47/34, A01N 47/36

(21) Anmeldenummer: **87111932.7**

(22) Anmeldetag: **18.08.87**

Die Akte enthält technische Angaben, die nach dem Eingang der Anmeldung eingereicht wurden und die nicht in dieser Patentschrift enthalten sind.

(54) **Chinolin-8-carbonsäurederivate.**

(30) Priorität: **21.08.86 DE 3628356**

(43) Veröffentlichungstag der Anmeldung:
**02.03.88 Patentblatt 88/09**

(45) Bekanntmachung des Hinweises auf die Patenterteilung:
**02.01.92 Patentblatt 92/01**

(84) Benannte Vertragsstaaten:
**CH DE FR GB IT LI**

(56) Entgegenhaltungen:
**EP-A- 0 060 429**
**EP-A- 0 104 389**
**EP-A- 0 132 714**

(73) Patentinhaber: **BASF Aktiengesellschaft**
**Carl-Bosch-Strasse 38**
**W-6700 Ludwigshafen(DE)**

(72) Erfinder: **Hagen, Helmut, Dr.**
**Max-Slevogt-Strasse 17e**
**W-6710 Frankenthal(DE)**
Erfinder: **Dupuis, Jacques, Dr.**
**Bannwasserstrasse 37**
**W-6700 Ludwigshafen(DE)**
Erfinder: **Eichenauer, Ulrich, Dr.**
**Paul-Ehrlich-Strasse 25 a**
**W-6000 Frankturt 70(DE)**
Erfinder: **Wuerzer, Bruno, Dr.**
**Ruedigerstrasse 13**
**W-6701 Otterstadt(DE)**
Erfinder: **Kohler, Rolf-Dieter, Dr.**
**Amselweg 3**
**W-6803 Edingen-Neckarhausen(DE)**
Erfinder: **Meyer, Norbert, Dr.**
**Dossenheimer Weg 22**
**W-6802 Ladenburg(DE)**

EP 0 257 518 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Chinolin-8-carbonsäurederivate der allgemeinen Formel I

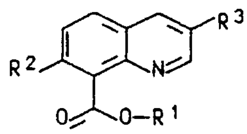

(I),

in der die Substituenten folgende Bedeutung haben:

- $R^1$ Wasserstoff, $C_1$-$C_4$-Alkyl, $C_2$-$C_4$-Alkenyl, ein Äquivalent eines Alkali- oder Erdalkalimetallkations,
- $R^2$ Fluor, Chlor oder Brom,
- $R^3$ Fluor, Brom, Jod, Rhodanid, die Nitro-, die Amino-, die Hydrazingruppe, $-N_2^{\oplus}Z^{\ominus}$, NHCHO,

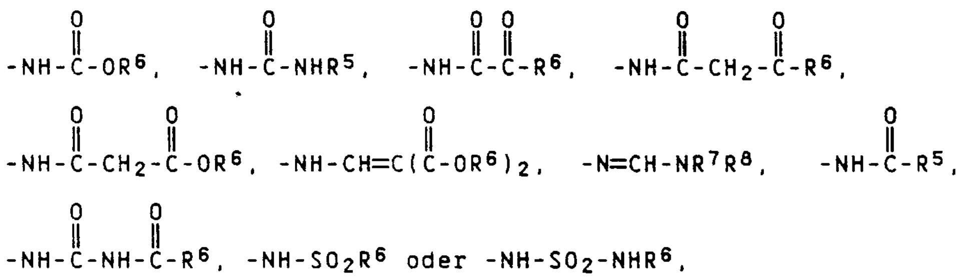

- Z $-Br^{\ominus}$, $BF_4^{\ominus}$, $PF_6^{\ominus}$ oder $SbF_6^{\ominus}$
- $R^5$ Wasserstoff,
  $C_1$-$C_4$-Alkyl, $C_1$-$C_4$-Hydroxyalkyl, $C_1$-$C_4$-Halogenalkyl, gegebenenfalls durch Halogen, $C_1$-$C_4$-Alkyl substituiertes Phenyl, $C_2$-$6_5$-Hydroxycarbonylalkyl, $C_3$-$C_8$-Alkoxycarbonylalkyl,
- $R^6$ $C_1$-$C_4$-Alkyl, $C_1$-$C_4$-Halogenalkyl, $C_2$-$C_5$-Alkylcarbonyl, $C_2$-$C_5$-Halogenalkylcarbonyl oder gegebenenfalls durch Halogen oder $C_1$-$C_4$-Alkyl substituiertes Phenyl,
- $R^7$, $R^8$ $C_1$-$C_4$-Alkyl.

Außerdem betrifft die Erfindung die Herstellung der Verbindungen I, ihre Verwendung als Herbizide, sowie Herbizide, die die Verbindungen I enthalten, und ein Verfahren zur Bekämpfung unerwünschten Pflanzenwuchses.

Aus der DE-OS 32 33 089 sind Chinolin-8-carbonsäurederivate als Herbizide bekannt, die sich von den Verbindungen I durch eine andere Substitution in 3-Stellung unterscheiden.

Der Erfindung lag die Aufgabe zugrunde, neue Verbindungen dieses Typs als Herbizide bereitzustellen; wobei diese nach Möglichkeit besser abbaubar sein sollten als die entsprechenden bekannten Verbindungen.

Demgemäß wurden die eingangs definierten neuen Chinolin-8-carbonsäurederivate I gefunden. Weiterhin wurden Verfahren zur Herstellung der Verbindungen I gefunden. Ferner wurde gefunden, daß die Verbindungen I sich gut als Herbizide und darüber hinaus aus z.B. als Zwischenprodukte zur Herstellung von Chinolin-8-carbonsäurederivaten, die in 3-Position fluoralkylsubstituiert sind, eignen.

Die Verbindungen I sind nach folgenden Methoden erhältlich:

a) Herstellung der Verbindungen I für den Fall, daß das Chinolin-Ringsystem und der Substituent $R^3$ über eine C-N-Bindung verknüpft sind

Ausgehend von Mucobromsäure kann das Natriumsalz des Nitromalondialdehyds (Organic Synthesis 32, 95 (1952)) hergestellt und zur Synthese der 3-Nitrochinolinderivate II (Chem. Rev. 60, 261 (1960)), analog zu den in J. Chem. Soc. 1948, 2024 und J. Org. Chem. 10, 76 (1945) gegebenen Vorschriften, mit entsprechend substituierten Anilin-Hydrochloriden im wäßrigen Medium und anschließender Cyclisierung unter Wasserabspaltung umgesetzt werden. Die Cyclisierung kann bei erhöhter Temperatur in einem sauren Verdünnungsmittel wie Polyphosphorsäure, Phosphoroxitrichlorid, Schwefelsäure, Essigsäure, Acetanhydrid oder einem inerten, hochsiedenden Lösungsmittel wie Sulfolan, Diphenylether, Biphenyl, Naphthalin, Dichlorbenzol oder deren Gemischen durchgeführt werden. Bevorzugt wird in Polyphosphorsäure bei Temperaturen zwischen 100 und 160 °C cyclisiert; besonders bevorzugt sind Temperaturen

zwischen 120 und 140°C.

Die anschließende Oxidation der Methylgruppe in 8-Position zur Carboxylgruppe kann ein- oder mehrstufig erfolgen. Bevorzugt wird die Bromierung der Methylgruppe mit anschließender Oxidation.

Dazu werden die Verbindungen II mit der Methylgruppe in 8-Stellung mit einem Halogenierungsmittel wie Brom, Chlor, N-Bromsuccinimid, N-Chlorsuccinimid, N-Chloracetamid, N-Bromacetamid, N-Chlorphthalimid oder N-Bromphthalimid in einem inerten Lösungsmittel bei erhöhter Temperatur umgesetzt, wobei die typischen Bedingungen einer Radikalreaktion (Anwesenheit von Radikalstarter, Belichtung) vorteilhaft sind.

Als inerte Lösungsmittel eignen sich perhalogenierte Kohlenwasserstoffe wie Tetrachlormethan, Hexachlorethan, Trifluortrichlorethan oder chlorierte Aromaten wie Chlorbenzol oder Dichlorbenzol. Die Reaktionstemperatur kann je nach Lösungsmittel zwischen 60 und 180°C gewählt werden; als Radikalstarter sind Azoverbindungen wie Azobis(isobuttersäurenitril) oder Peroxide wie Dibenzoylperoxid geeignet. Zur Belichtung genügt diffuses Sonnenlicht, es kann durch Glühlampen verstärkt werden.

Bevorzugt sind Reaktionsbedingungen, bei denen Chlorbenzol als Reaktionsmedium, Temperaturen zwischen 80 und 140°C, besonders bevorzugt Temperaturen zwischen 110 und 140°C, und Azobis(isobuttersäurenitril) als Radikalstarter verwendet werden.

Die Oxidation der $CH_2Br$-Gruppe in 8-Position zur Carboxylgruppe kann durch Umsetzung mit Salpetersäure in Schwefelsäure, bevorzugt bei 100 bis 140°C, erfolgen.

Soll $R^1$ verändert werden, beispielsweise zu $C_1$-$C_4$-Alkyl, geschieht dies bevorzugt auf dieser Stufe, beispielsweise durch alkylierende Veresterung mit Dialkylsulfaten oder Alkylhalogeniden oder durch eine andere an sich bekannte Methode zur Estersynthese aus Carbonsäuren.

Die so erhaltenen 3-Nitrochinolin-8-carbonsäurederivate der Formel Ie, $R^1$ = Wasserstoff oder $C_1$-$C_4$-Alkyl kann man zu den 3-Aminochinolinderivaten If reduzieren, in dem man eine der in Houben-Weyl, "Methoden der organischen Chemie", Bd. 11/1, Kap. IV.2 angegebenen Standardmethoden zur katalytischen oder chemischen Reduktion anwendet. Bevorzugt wird die katalytische Hydrierung, wobei Edelmetallkatalysatoren wie Platin, Palladium, Rhodium oder Nickel, gegebenenfalls auf inerten Trägermaterialien wie Aktivkohle, Kieselgel oder Aluminiumoxid, in Verdünnungsmitteln wie $C_1$-$C_6$-Alkoholen, Carbonsäureestern dieser Alkohole, Carbonsäuren wie Essigsäure oder Propionsäure, cyclischen Ethern wie Dioxan oder Tetrahydrofuran, Amiden wie Dimethylformamid oder N-Methylpyrrolidon oder deren Gemischen verwendet werden können. Reaktionstemperaturen zwischen 20 und 100°C und Reaktionsdrucke zwischen 1 und 50 bar eignen sich für diese Reaktion. Bevorzugt wird die Reaktion mit Raney-Nickel in Gemischen aus Dimethylformamid und iso-Butanol bei Normaldruck zwischen 40 und 60°C durchgeführt. Die Reaktionssequenz sei durch das folgende Schema verdeutlicht:

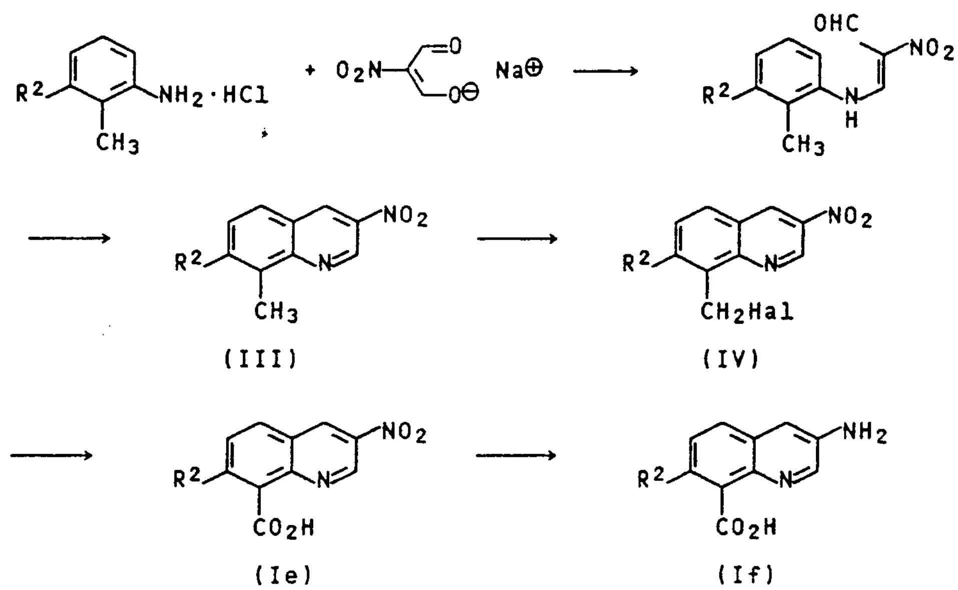

(III) (IV) (Ie) (If)

Eine weitere Methode zur Herstellung der 3-Aminochinolinderivate If geht von den 3-Acylchinolinderivaten Ic und den daraus herstellbaren Oximen Id aus, die nach Reinisolierung oder in situ einer Beckmann-Umlagerung unterworfen werden können.

Die Beckmann-Umlagerung kann nach einer der in Houben-Weyl, "Methoden der organischen Chemie",

Bd. 11/1, Kap. VIIc, angeführten Standardmethoden oder in einer organischen Carbonsäure wie Ameisensäure, Essigsäure oder Propionsäure unter Zusatz eines stark sauren Katalysators wie konzentrierter Schwefelsäure, Trifluoressigsäure, Trifluormethansulfonsäure bei erhöhter Temperatur ausgeführt werden. Bevorzugt sind Ameisensäure als Medium und Trifluormethansulfonsäure als Katalysator, wobei die Reaktionstemperatur zwischen 90 °C und der jeweiligen Rückflußtemperatur liegt.

Die Hydrolyse der so erhaltenen acylierten 3-Aminochinolinderivate V

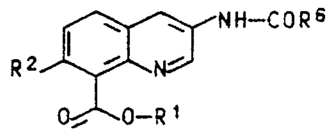

(V)

kann wie beispielsweise in Houben-Weyl, Bd. 11/1, Kap. VIIIa angegeben, erfolgen.

Die 3-Aminochinolinderivate If können nach gängigen Methoden, wie sie in Houben-Weyl, Bd. 11/2, Kap. BIa beschrieben werden, acyliert werden. Bevorzugte Verfahren sind:

a) die Umsetzung mit Carbonsäurechloriden in inerten Lösungsmitteln wie chlorierten Kohlenwasserstoffen wie Methylenchlorid, Tetrachlormethan, Ethern wie Tetrahydrofuran, Dioxan, Diethylether oder Dimethoxyethan oder Aromaten wie Toluol oder Chlorbenzol in Gegenwart einer Hilfsbase wie Triethylamin oder Pyridin bei -10 bis +40 °C; besonders bevorzugt werden Dimethoxyethan als Lösungsmittel, Pyridin und Triethylamin als Basen bei 0 bis +20 °C,

b) die Umsetzung mit Carbonsäureanhydriden in einem Verdünnungsmittel, bevorzugt in der korrespondierenden Carbonsäure, bei erhöhter Temperatur besonders bevorzugt unter Rückfluß der Carbonsäure.

Zur Formylierung kann ohne weiteres Hilfsmittel in Ameisensäure zum Rückfluß erhitzt werden.

Zur Diazotierung kann nach einem in Houben-Weyl, Bd. 10/3, Kap. AI angegebenen Verfahren mit Natriumnitrit umgesetzt werden. Bevorzugt ist die Reaktion in einer Carbonsäure wie Essigsäure oder Propionsäure oder Gemischen aus diesen in Gegenwart mindestens zweier Äquivalente einer starken Säure wie Chlorwasserstoffsäure, Bromwasserstoffsäure, Schwefelsäure oder einer Komplexsäure wie $HBF_4$, $HPF_6$, $HSbF_6$ bei Temperaturen zwischen -10 und +5 °C. Soll das Diazoniumsalz der Formel Ig isoliert werden, so kann eine Komplexsäure mit stabilisierendem Gegenion Z⊖ verwendet werden.

Die Reduktion des Diazoniumsalzes (Houben-Weyl, Bd. 10/2, Kap. 5 $A_1$I) liefert Hydrazinderivate der Formel Ij

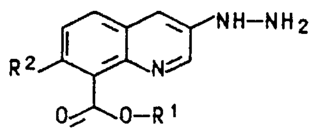

(Ij)

Die Substitution der Diazoniumgruppe in 3-Stellung des Chinolinderivates Ig durch Halogene wie F, Br und I oder Pseudohalogene wie Rhodanid, ist allgemein bekannt (Houben-Weyl, Bd. 5/3 Kap. Alk (F), Bd. 5/4, Kap. $A_2$IVc (Br), Bd. 5/4 Kap. $A_3$IVc (J) und Bd. 9, Kap. 24 IV (SCN)).

Zur Synthese des 3-Fluorchinolins der Formel Ih, kann das Diazoniumtetrafluoroboratder Formel Ig, in einem inerten Lösungsmittel wie Toluol oder Chlorbenzol erhitzt werden. Das 3-Bromchinolin kann aus der Lösung des Diazoniumsalzes der Formel Ig, ($R^3$ = $N_2$⊕Br⊖) durch Erwärmen auf 20 bis 60 °C hergestellt werden, während 3-Iodchinoline und 3-Thiocyanatochinolin der Formel Ih ($R^3$ = I bzw. SCN) durch Umsetzung mit Alkalimetallsalzen der Iod- bzw. Rhodanwasserstoffsäure in wäßrigem Medium bei 0 bis 40 °C hergestellt werden können.

Im einzelnen haben die Substituenten folgende Bedeutung:

$R^1$ - Wasserstoff
- $C_1$-$C_4$-Alkyl, beispielsweise Methyl, Ethyl, n-Propyl, iso-Propyl, n-Butyl, iso-Butyl, tert.-Butyl, besonders Methyl und Ethyl
- $C_2$-$C_4$-Alkenyl, z.B. Vinyl oder Allyl, besonders Allyl,

$R^2$ - Fluor, Chlor oder Brom, vorzugsweise Fluor und Chlor,

$R^3$ - Fluor, Brom oder Jod,
- SCN,
- $NO_2$,

- $NH_2$,
- $N_2^{\oplus}Z^{\ominus}$, beispielsweise $N_2^{\oplus}BF_4^{\ominus}$, $N_2^{\oplus}PF_6^{\ominus}$ oder $N_2^{\oplus}SbF_6^{\ominus}$,
- NHCHO,
- $NH-NH_2$,
- $NH-\overset{O}{\overset{\|}{C}}-R^5$,

beispielsweise NHCO, $NHCOCH_3$, $NHCOC_2H_5$, $NHCOCH=CH_2$, $NHCOCH_2Cl$, $NHCOCH_2CH_2Cl$, $NHCOCH(CH_3)2$, $NHCOC(CH_3)_3$, und $NHCOCF_3$ bevorzugt sind $NHCOCH_3$ und $NHCOC_2H_5$,

- $NH-\overset{O}{\overset{\|}{C}}-NHR^6$, beispielsweise $NHCO-NH-CH_3$, $NHCO-NH-C_2H_5$, $NHCONH-C_3H_7$, vorzugsweise $NHCO-NH-CH(CH_3)_2$, $NHCO-NH-C_6H_5$, $NHCO-NH-COCl_3$,
- $NH-\overset{O}{\overset{\|}{C}}-\overset{O}{\overset{\|}{C}}-OR^6$, beispielsweise $NHCO-CO_2CH_3$, $NHCO-CO_2C_3H_7$, vorzugsweise $NHCO-CO_2C_2H_5$,
- $NH-\overset{O}{\overset{\|}{C}}-CH_2-\overset{O}{\overset{\|}{C}}-R^6$, beispielsweise $NHCO-CH_2-COC_2H_5$, $NH-CO-CH_2-CO-n-C_4H_9$, vorzugsweise $NHCO-CH_2-COCH_3$,
- $NH-\overset{O}{\overset{\|}{C}}-OR^6$, beispielsweise $NH-\overset{O}{\overset{\|}{C}}-OCH_3$, $NH-\overset{O}{\overset{\|}{C}}-OC_2H_5$, $NH-\overset{O}{\overset{\|}{C}}-OC_3H_7$, $NH-\overset{O}{\overset{\|}{C}}-OC_4H_9$, bevorzugt sind $NH-\overset{O}{\overset{\|}{C}}-OCH_3$, $NH-\overset{O}{\overset{\|}{C}}-OC_2H_5$ .
- $NH-\overset{O}{\overset{\|}{C}}-CH_2-\overset{O}{\overset{\|}{C}}-OR^6$, beispielsweise $NHCOCH_2COCH_3$, $NHCOCH_2CO_2CH_3$ oder $NHCOCH_2CO_2C_2H_5$,
- $NH-CH=C(\overset{O}{\overset{\|}{C}}-OR^6)_2$, z.B. $NH-CH=C(\overset{O}{\overset{\|}{C}}-OCH_3)_2$, $NH-CH=C(\overset{O}{\overset{\|}{C}}-OC_2H_5)_2$,
- $N=CH-NR^7R^8$, z.B. $N=CH-N(CH_3)_2$, $N=CH-N(C_2H_5)_2$, $N=CH-N(CH_3)C_2H_5$, bevorzugt ist $N=CH-N(CH_3)_2$,
- $NH-\overset{O}{\overset{\|}{C}}-NH-\overset{O}{\overset{\|}{C}}-R^6$, z.B. $NH-\overset{O}{\overset{\|}{C}}-NH-\overset{O}{\overset{\|}{C}}-CH_3$, $NH-\overset{O}{\overset{\|}{C}}-NH-\overset{O}{\overset{\|}{C}}-C_2H_5$, und $NH-\overset{O}{\overset{\|}{C}}-NH-\overset{O}{\overset{\|}{C}}-CH(CH_3)_2$
- $NH-SO_2R^6$, z.B. $NH-SO_2CH_3$, $NH-SO_2C_2H_5$, $NH-SO_2C_3H_7$, $NH-SO_2C_4H_9$ und $NH-SO_2-C_6H_4CH_3$,
- $NH-SO_2NHR^6$, z.B. $NH-SO_2NHCH_3$, $NH-SO_2NHC_2H_5$, vorzugsweise $NH-SO_2NHCH_3$ und $NH-SO_2NHCH(CH_3)_2$.

In der nachstehenden Tabelle A sind geeignete Verbindungen der allgemeinen Formel I übersichtlich

aufgeführt; in den weiter unten folgenden Tabellen 1 bis 7 sind die Verbindungen, die hergestellt wurden, mit ihren Schmelzpunkten oder anderen physikalischen Angaben versehen.

Tabelle A - Beispiele für geeignete Verbindungen I sind:

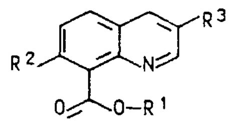

| Verbindung-Nr. | $R^1$ | $R^2$ | $R^3$ |
|---|---|---|---|
| 85 | H | Cl | $NO_2$ |
| 86 | $CH_3$ | Cl | $NO_2$ |
| 87 | H | F | $NO_2$ |
| 88 | $CH_3$ | F | $NO_2$ |
| 89 | H | Cl | $NH_2$ |
| 90 | $CH_3$ | Cl | $NH_2$ |
| 91 | H | F | $NH_2$ |
| 92 | $CH_3$ | F | $NH_2$ |
| 93 | H | Cl | NHCHO |
| 94 | H | Cl | $NHCOCH_3$ |
| 95 | $CH_3$ | Cl | $NHCOCH_3$ |
| 96 | H | Cl | $NHCOCH_2Cl$ |
| 97 | H | Cl | $NHCOCH_2CH_3$ |
| 98 | H | Cl | $NHCO_2CH_3$ |
| 99 | H | Cl | $NHCOCO_2C_2H_5$ |
| 100 | H | Cl | $NHCOCF_3$ |
| 101 | H | Cl | $NHCOCH_2COCH_3$ |
| 102 | H | Cl | $NHCOCH_2CO_2CH_3$ |
| 103 | H | Cl | $NHCONHCH(CH_3)_2$ |
| 104 | H | Cl | $NHCONHC_6H_5$ |
| 105 | H | Cl | $NHCONHCOCCl_3$ |
| 106 | H | Cl | $NHCONHCOCH_3$ |
| 107 | H | Cl | $NHSO_2NHCH_3$ |
| 108 | H | Cl | $NHSO_2NHCH(CH_3)_2$ |
| 109 | H | Cl | $NHCH=C(CO_2C_2H_5)_2$ |

| Verbindung-Nr. | $R^1$ | $R^2$ | $R^3$ |
|---|---|---|---|
| 110 | H | Cl | $N{=}CH{-}N(CH_3)_2$ |
| 111 | H | Cl | $N_2^{\oplus}BF_4^{\ominus}$ |
| 112 | $CH_3$ | Cl | $N_2^{\oplus}BF_4^{\ominus}$ |
| 113 | H | Cl | F |
| 114 | $CH_3$ | Cl | F |
| 115 | H | F | F |
| 116 | $CH_3$ | F | F |
| 117 | H | Cl | J |
| 118 | $CH_3$ | Cl | J |
| 119 | H | Cl | SCN |
| 120 | $CH_3$ | Cl | SCN |
| 121 | H | F | SCN |
| 122 | $CH_3$ | F | SCN |
| 123 | H | Br | F |
| 124 | $CH_3$ | Br | F |
| 125 | H | Br | Br |
| 126 | $CH_3$ | Br | Br |
| 127 | H | Cl | Br |
| 128 | $CH_3$ | Cl | Br |

Die Chinolin-8-carbonsäurederivate der Formel I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgier-mittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol oder Nonylphenol, Alkylphenolpolyglykolether, Tributylphenylpolyglykolether, Alkylarylpolyetheralkohole, Isotridecylalkohol,

Fettalkoholethylenoxid-Kondensate, ethoxyliertes Rizinusöl, Polyoxyethylenalkylether, ethoxyliertes Polyoxypropylen, Laurylalkoholpolyglykoletheracetat, Sorbitester, Lignin, Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff.

Die Applikation kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

In Anbetracht des erfaßbaren Wirkungsspektrums zur Unkrautbekämpfung, der Verträglichkeit der Kulturpflanzen oder der erwünschten Beeinflussung des Wachstums derselben sowie angesichts der Vielfalt der Applikationsmethoden können die erfindungsgemäßen Verbindungen I je nach Substitutionsmuster in einer großen Zahl von Kulturpflanzen eingesetzt werden.

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |

| Botanischer Name | Deutscher Name |
|---|---|
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochua | Zuckerhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Chinolin-8-carbonsäurederivate der Formel I sowohl untereinander als auch mit Vertretern anderer herbizider oder wachstumsregulierender Wirkstoffgruppen gemischt und gemeinsam ausgebracht werden.

Außerdem kann es von Nutzen sein, die Chinolin-8-carbonsäurederivate der Formel I bzw. sie enthaltende herbizide Mittel allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Herstellungsbeispiele

Beispiel 1
Herstellung von 7-Halogen-3-nitro-chinolin-8-carbonsäuren

450 mmol eines 7-Halogen-8-methyl-3-nitro-chinolins wurden mit 1400 mmol N-Bromsuccinimid und 5 g Azobis(isobuttersäurenitril) in 500 ml Chlorbenzol 3,5 Stunden unter Rückfluß erhitzt und wie üblich aufgearbeitet.

100 mmol dieser Verbindung wurden in 120 ml 70 % Schwefelsäure suspendiert. Bei 120 °C wurden 30 g 65 % Salpetersäure eingetropft und nach 2 Stunden bei 120 °C auf Eis gegossen und wie üblich aufgearbeitet.

Die Ergebnisse sind in Tabelle 1 zusammengestellt.

Beispiel 2
Herstellung von 3-Amino-7-halogen-chinolin-8-carbonsäuren

300 mmol einer 7-Halogen-3-nitro-chinolin-8-carbonsäure wurden mit 25 g Raney-Nickel bei 60 °C in 300 ml Dimethylformamid hydriert. Nach Aufnahme der berechneten Menge Wasserstoff wurde Aktivkohle zugesetzt und wie üblich aufgearbeitet.

Die Ergebnisse sind in Tabelle 1 zusammengestellt.

Beispiel 3
Herstellung von 7-Halogen-3-nitro- bzw. -amino-chinolin-8-carbonsäuremethylester

200 mmol einer 7-Halogen-3-nitro- bzw. -amino-chinolin-8-carbonsäure wurden in 250 ml Aceton suspendiert. Anschließend wurden 27,7 g Dimethylsulfat und 27,7 g Natriumhydrogencarbonat zugegeben, das Gemisch 6 Stunden unter Rückfluß gerührt und wie üblich aufgearbeitet.

Die Ergebnisse sind in Tabelle 1 zusammengestellt:

Tabelle 1

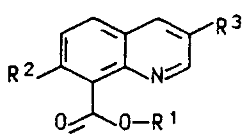

| Verbindung-Nr. | $R^1$ | $R^2$ | $R^3$ | Fp.[°C] |
|---|---|---|---|---|
| 85 | H | Cl | $NO_2$ | 150 |
| 86 | $CH_3$ | Cl | $NO_2$ | 175 |
| 87 | H | F | $NO_2$ | |
| 88 | $CH_3$ | F | $NO_2$ | |
| 89 | H | Cl | $NH_2$ | 190 |
| 90 | $CH_3$ | Cl | $NH_2$ | 140 |
| 91 | H | F | $NH_2$ | |
| 92 | $CH_3$ | F | $NH_2$ | |

Beispiel 4
Herstellung von in 3-Position substituierten 3-Amino-7-halogen-chinolin-8-carbonsäuren und deren Ester

20 mmol einer 3-Amino-7-halogen-chinolin-8-carbonsäure oder deren Ester wurden in 50 ml Dimethoxyethan suspendiert und mit 4 ml Pyridin versetzt. Anschließend wurden bei 0 °C 27 mmol des entsprechenden Acetylierungsmittels zugetropft. Nach 7 Stunden bei 25 °C wurde auf Eis gegeben und wie üblich aufgearbeitet.

Die Ergebnisse sind in Tabelle 2 zusammengestellt.

Tabelle 2

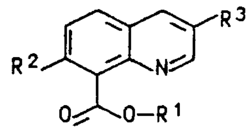

| Verbindung-Nr. | $R^1$ | $R^2$ | $R^3$ | Fp. [°C] |
|---|---|---|---|---|
| 93 | H | Cl | NHCHO | 228-230 |
| 94 | H | Cl | $NHCOCH_3$ | 185 |
| 95 | $CH_3$ | Cl | $NHCOCH_3$ | 254 |
| 96 | H | Cl | $NHCOCH_2Cl$ | 240 |
| 97 | H | Cl | $NHCOCH_2CH_3$ | 263 |
| 98 | H | Cl | $NHCO_2CH_3$ | > 360 |
| 99 | H | Cl | $NHCOCO_2C_2H_5$ | 228 |
| 100 | H | Cl | $NHCOCF_3$ | 259 |
| 101 | H | Cl | $NHCOCH_2COCH_3$ | 230 |
| 102 | H | Cl | $NHCOCH_2CO_2CH_3$ | |
| 103 | H | Cl | $NHCONHCH(CH_3)_2$ | |
| 104 | H | Cl | $NHCONHC_6H_5$ | |
| 105 | H | Cl | $NHCONHCOCCl_3$ | 295 |
| 106 | H | Cl | $NHCONHCOCH_3$ | |
| 107 | H | Cl | $NHSO_2NHCH_3$ | |
| 108 | H | Cl | $NHSO_2NHCH(CH_3)_2$ | 100 |
| 109 | H | Cl | $NHCH{=}C(CO_2C_2H_5)_2$ | 238 |
| 110 | H | Cl | $N{=}CH{-}N(CH_3)_2$ | amorph |

Beispiel 5
Herstellung von 7-Halogen-3-diazoniumtetrafluorborato-chinolin-8-carbonsäuren und deren Ester

15 mmol einer 3-Amino-7-halogen-chinolin-8-carbonsäure oder deren Ester wurden in 30 ml Propionsäure und 5 ml 50 %iger Tetrafluorborsäure suspendiert. Bei -10 °C wurden danach 1,5 g Natriumnitrit langsam eingetragen. Man rührte 1 Stunde bei 0 °C, goß in Eiswasser und arbeitete wie üblich auf.

Beispiel 6
Herstellung von 7-Halogen-3-fluor-chinolin-8-carbonsäure und deren Ester

10 mmol einer 7-Halogen-3-diazoniumtetrafluoroborato-chinolin-8-carbonsäure und deren Ester wurden in 20 ml Toluol 1 Stunde unter Rückfluß erhitzt, das Toluol vom sich bildenden Rückstand abgegossen, 20 ml 5 %ige wäßrige Natronlauge zugegeben, 1 Stunde unter Rückfluß erhitzt und wie üblich aufgearbeitet.

Die Ergebnisse sind in Tabelle 3 zusammengestellt.

Beispiel 7
Herstellung von 3-Brom-7-halogen-chinolin-8-carbonsäuren und deren Ester

20 mmol 3-Amino-7-halogen-chinolin-8-carbonsäure oder deren Ester wurden in einem Gemisch aus 25 ml Eisessig und 25 ml Propionsäure mit 7,9 g Nitrosylschwefelsäure bei 0°C diazotiert, das Gemisch 30 min nachgerührt und bei 40°C zu einer Suspension von Kupfer-I-bromid, hergestellt aus 5,7 g Kupfer-II-sulfat in 24 ml Wasser und 3,6 g Kaliumbromid, 1,45 g Kupferpulver in 9 ml Wasser und 13,2 ml 48 % Bromwasserstoff-Lösung, gegeben. Es wurde 30 min bei 40°C nachgerührt, abgesaugt, mit Wasser gewaschen und der Rückstand 2 h mit konz. Ammoniak gerührt und wie üblich aufgearbeitet.

Die Ergebnisse sind in Tabelle 3 zusammengestellt.

Tabelle 3

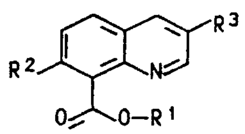

| Verbindung-Nr. | $R^1$ | $R^2$ | $R^3$ | Fp. [°C] | $^1$H-NMR [ppm] |
|---|---|---|---|---|---|
| 111 | H | Cl | $N_2^{\oplus}BF_4^{\ominus}$ | nicht bestimmt | |
| 112 | $CH_3$ | Cl | $N_2^{\oplus}BF_4^{\ominus}$ | nicht bestimmt | δ = 4,31 (s), 8,12 (d), 8,43 (d), 9,95 (s) in $CF_3COOH$ |
| 113 | H | Cl | F | 218-220 | |
| 114 | $CH_3$ | Cl | F | | |
| 115 | H | F | F | | |
| 116 | $CH_3$ | F | F | | |
| 117 | H | Cl | J | 135 | |
| 118 | $CH_3$ | Cl | J | | |
| 119 | H | Cl | SCN | | |
| 120 | $CH_3$ | Cl | SCN | | |
| 121 | H | F | SCN | | |
| 122 | $CH_3$ | F | SCN | | |
| 123 | H | Br | F | | |
| 124 | $CH_3$ | Br | F | | |
| 125 | H | Br | Br | | |
| 126 | $CH_3$ | Br | Br | | |
| 127 | H | Cl | Br | > 270 | |
| 128 | $CH_3$ | Cl | Br | 117-120 | |

Anwendungsbeispiele

Folgende Pflanzen wurden auf ihr Verhalten gegenüber einigen der Verbindungen I untersucht:

| Lateinischer Name | Deutscher Name |
|---|---|
| Avena sativa | Hafer |
| Centaurea cyanus | Kornblume |
| Echinochloa crus-galli | Hühnerhirse |
| Galium aparine | Klettenlabkraut |
| Ipomoea spp. | Prunkwinkearten |
| Triticum aestivum | Weizen |
| Veronica spp. | Ehrenpreisarten |
| Zea mays | Mais |

Die herbizide Wirkung der Chinolin-8-carbonsäurederivate der Formel I auf das Wachstum der Testpflanzen wurde durch folgende Gewächshausversuche gezeigt.

Als Kulturgefäße dienten Plastikblumentöpfe mit 300 $cm^3$ Inhalt und lehmigem Sand mit etwa 3,0 % Humus als Subtrat. Bei Soja wurde etwas Torfmull zugesetzt, um einen besseren Stand zu erzielen. Die Samen der Testpflanzen wurden nach Arten getrennt flach eingesät.

Zum Zwecke der Nachauflaufbehandlung wurden entweder direkt gesäte oder in den gleichen Gefäßen aufgewachsene Pflanzen ausgewählt oder erst als Keimpflanzen getrennt angezogen und einige Tage vor der Behandlung in die Versuchsgefäße verpflanzt.

Je nach Wuchsform wurden die Testpflanzen bei einer Wuchshöhe von 3 bis 15 cm dann mit den in Wasser als Verteilungsmittel suspendierten oder emulgierten Wirkstoffen, die durch fein verteilende Düsen gespritzt wurden behandelt. Die Aufwandmengen für die Nachauflaufbehandlung variierten. Sie betrugen 1,0 bis 3,0 kg Wirkstoff/ha.

Die Versuchsgefäße wurden im Gewächshaus aufgestellt, wobei für wärmeliebende Arten wärmere Bereich (20 bis 35 °C) und für solche gemäßigter Klimate 10 bis 20 °C bevorzugt wurden. Die Versuchsperiode erstreckte sich über 2 bis 4 Wochen. Während dieser Zeit wurden die Pflanzen gepflegt und ihre Reaktion auf die einzelnen Behandlungen ausgewertet.

Mit 1,0 kg Wirkstoff/ha ließen sich sowohl im Nachauflauf- als auch im Vorauflaufverfahren mit Verbindung 127 breitblättrige unerwünschte Pflanzen wie Galium aparine oder Veronica spp. bekämpfen bei gleichzeitiger guter Verträglichkeit für Weizen und Mais.

Mit 3,0 kg Wirkstoff/ha im Nachauflaufverfahren konnnten Centaurea cyanus, Echinochloa crus-galli und Ipomoea spp. bei guter Verträglichkeit in Hafer mit Verbindung 127 bekämpft werden.

## Patentansprüche

**1.** Chinolin-8-carbonsäurederivat der allgemeinen Formel I

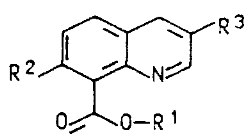

(I),

in der die Substituenten folgende Bedeutung haben:

- $R^1$ Wasserstoff, $C_1$-$C_4$-Alkyl, $C_2$-$C_4$-Alkenyl, ein Äquivalent eines Alkali- oder Erdalkalimetallkations,
- $R^2$ Fluor, Chlor oder Brom,
- $R^3$ Fluor, Brom, Jod, Rhodanid, die Nitro-, die Amino-, die Hydrazingruppe, $-N_2^{\oplus}Z^{\ominus}$, NHCHO,

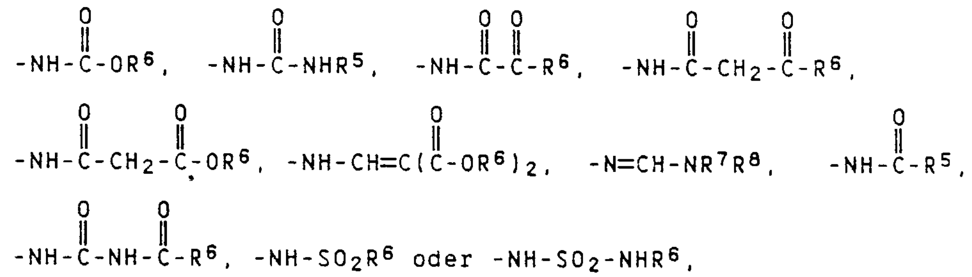

| | |
|---|---|
| Z | $-Br^{\ominus}$, $BF_4^{\ominus}$, $PF_6^{\ominus}$ oder $SbF_6^{\ominus}$ |
| $R^5$ | Wasserstoff, |
| | $C_1$-$C_4$-Alkyl, $C_1$-$C_4$-Hydroxyalkyl, $C_1$-$C_4$-Halogenalkyl, gegebenenfalls durch Halogen, $C_1$-$C_4$-Alkyl substituiertes Phenyl, $C_2$-$C_5$-Hydroxycarbonylalkyl, $C_3$-$C_8$-Alkoxycarbonylalkyl, |
| $R^6$ | $C_1$-$C_4$-Alkyl, $C_1$-$C_4$-Halogenalkyl, $C_2$-$C_5$-Alkylcarbonyl, $C_2$-$C_5$-Halogenalkylcarbonyl oder gegebenenfalls durch Halogen oder $C_1$-$C_4$-Alkyl substituiertes Phenyl, |
| $R^7$, $R^8$ | $C_1$-$C_4$-Alkyl. |

**2.** Verfahren zur Herstellung der Chinolin-8-carbonsäurederivate I des Anspruchs 1, dadurch gekennzeichnet, daß man

a) für den Fall, daß das Chinolin-Ringsystem und der Substituent $R^3$ über eine C-N-Bindung verknüpft sind, das Nitromalondialdehyd-Salz,

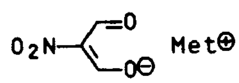

mit einem 3-Halogen-2-methylanilin-Hydrochlorid

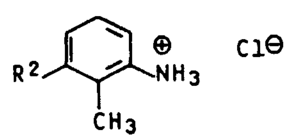

in an sich bekannter Weise umsetzt und zur Cyclisierung mit einer Säure oder einem Anhydrid in ebenfalls an sich bekannter Weise zu der Verbindung III

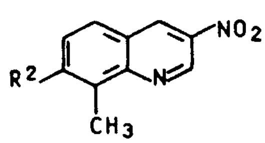 (III)

umsetzt,
diese durch Halogenierung in an sich bekannter Weise zu der Verbindung IV

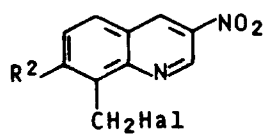

(IV)

umsetzt,
diese durch Oxidation ebenfalls in an sich bekannter Weise zu der Verbindung Ie

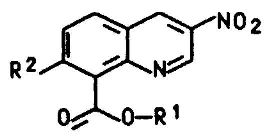

(Ie)

umsetzt,
auf dieser Stufe gegebenenfalls Variationen von $R^1$ vornimmt,
die Verbindung Ie in an sich bekannter Weise durch katalytische Hydrierung zu der Aminoverbindung If

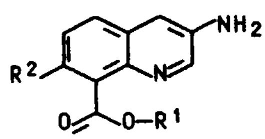

(If)

umsetzt,
diese in an sich bekannter Weise acyliert oder in ebenfalls an sich bekannter Weise zu der Verbindung Ig

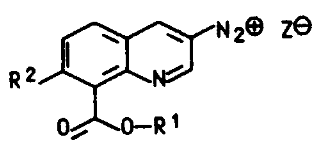

(Ig)

diazotiert, und
diese in an sich bekannter Weise zu einer in 3-Position Fluor, Brom, Jod oder SCN tragenden Verbindung Ih

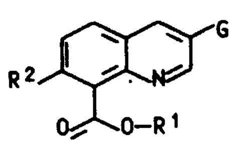

(Ih)

G = F, Br, J, SCN

umsetzt.

**3.** 3-Brom-7-chlor-chinolin-8-carbonsäuremethylester.

**4.** Verwendung eines Chinolin-8-carbonsäurederivats I gemäß Anspruch 1 als Herbizid.

**5.** Herbizid, enthaltend eine Verbindung der Formel I in wirksamer Menge neben hierfür üblichen Trägerstoffen.

**6.** Herbizid nach Anspruch 4, dadurch gekennzeichnet, daß es 0,1 bis 95 Gew.% eines Chinolin-8-carbonsäurederivates der Formel I gemäß Anspruch 1 enthält.

7. Verfahren zur Bekämpfung unerwünschten Pflanzenwuchses, dadurch gekennzeichnet, daß man die unerwünschten Pflanzen und/oder die von unerwünschtem Pflanzenwachstum freizuhaltende Flächen mit einer herbizid wirksamen Menge eines Chinolin-8-carbonsäurederivates I gemäß Anspruch 1 behandelt.

**Claims**

1. A quinoline-8-carboxylic acid derivative of the formula I

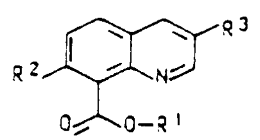

(I),

where
$R^1$ is hydrogen, $C_1$-$C_4$-alkyl, $C_2$-$C_4$-alkenyl, one equivalent of an alkali metal or alkaline earth metal cation,
$R^2$ is fluorine, chlorine or bromine,
$R^3$ is fluorine, bromine, iodine, thiocyanate, nitro, amino, the hydrazine group, $-N_2^{\oplus}Z^{\ominus}$, NHCHO,

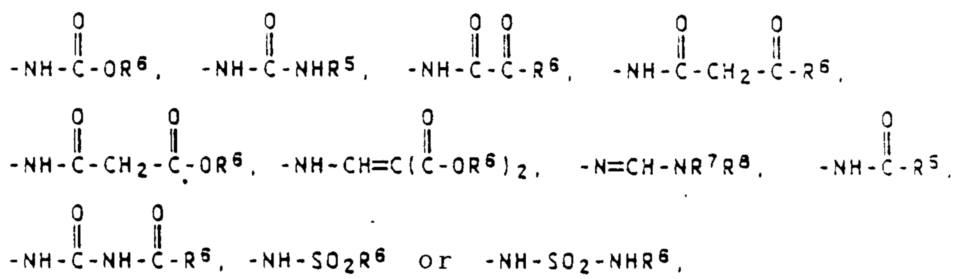

or

Z is $-Br^{\ominus}$, $BF_4^{\ominus}$, $PF_6^{\ominus}$ or $SbF_6^{\ominus}$
$R^5$ is hydrogen, $C_1$-$C_4$-alkyl, $C_1$-$C_4$-hydroxyalkyl, $C_1$-$C_4$-haloalkyl, unsubstituted, halogen-substituted or $C_1$-$C_4$-alkyl-substituted phenyl, $C_2$-$C_5$-hydroxycarbonylalkyl or $C_3$-$C_8$-alkoxycarbonylalkyl,
$R^6$ is $C_1$-$C_4$-alkyl, $C_1$-$C_4$-haloalkyl, $C_2$-$C_5$-alkylcarbonyl, $C_2$-$C_5$-haloalkylcarbonyl or unsubstituted, halogen-substituted or $C_1$-$C_4$-alkyl-substituted phenyl, and
$R^7$ and $R^8$ are each $C_1$-$C_4$-alkyl.

2. A process for preparing the quinoline-8-carboxylic acid derivative I of claim 1, which comprises a) - if the quinoline ring system and $R^3$ are linked via a C-N bond - reacting the nitromalodialdehyde salt

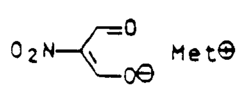

with a 3-halo-2-methylaniline hydrochloride

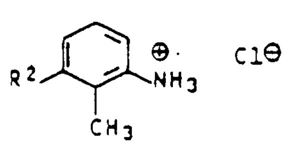

in a conventional manner and cyclizing the product, again in a conventional manner, with an acid or an anhydride to give a compound III

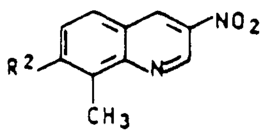

(III)

halogenating in a conventional manner to give a compound IV

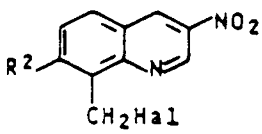

(IV)

oxidizing, again in a conventional manner, to give a compound Ie

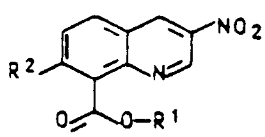

(Ie)

varying $R^1$ if desired at this stage; catalytically hydrogenating compound Ie in a conventional manner to give an amino compound If

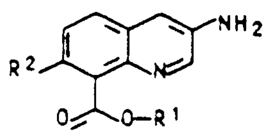

(If)

acylating or diazotizing in a conventional manner to give a compound Ig

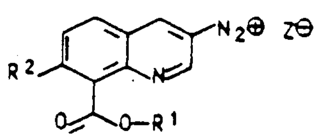

(Ig)

and converting in a conventional manner to give a compound Ih bearing fluorine, bromine, iodine or SCN in the 3-position

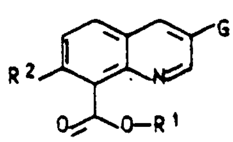

(Ih)

G = F, Br, I, SCN

3. Methyl 3-bromo-7-chloroquinoline-8-carboxylate.

4. The use of a quinoline-8-carboxylic acid derivative I as claimed in claim 1 as a herbicide.

5. A herbicide containing an effective amount of a compound of the formula I and conventional carriers.

6. A herbicide as claimed in claim 5, containing from 0.1 to 95% by weight of a quinoline-8-carboxylic acid derivative of the formula I as claimed in claim 1.

7. A method for controlling undesirable plant growth, wherein the undesirable plants and/or the areas to be kept free from undesiable plant growth are treated with a herbicidally effective amount of a quinoline-8-carboxylic acid derivative I as claimed in claim 1.

## Revendications

1. Dérivé d'acide quinoléine-8-carboxylique de la formule générale I

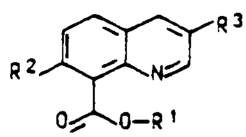

(I)

dans laquelle les substituants ont la signification suivante

- $R^1$, hydrogène, alkyle en $C_1$-$C_4$, alcényle en $C_2$-$C_4$, un équivalent d'un cation alcalin ou alcalino-terreux
- $R^2$, fluor, chrome ou brome
- $R^3$, fluor, brome, iode, sulfocyanure, les groupes nitro, amino, hydrazine, $-N_2^{\oplus}Z^{\ominus}$, NHCHO,

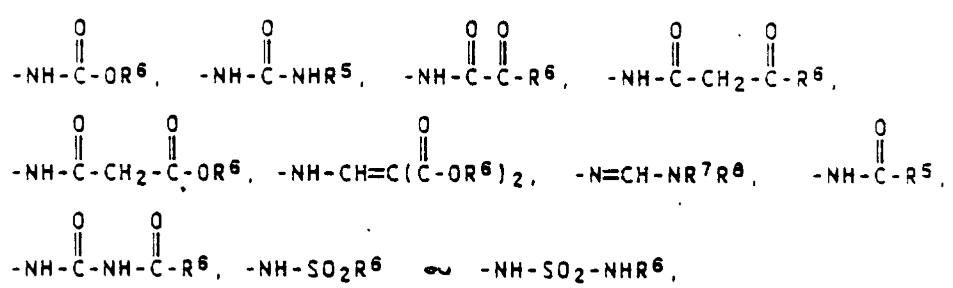

- Z, $-Br^{\ominus}$, $BF_4^{\ominus}$, $PF_6^{\ominus}$ ou $SbF_6^{\ominus}$
- $R^5$, hydrogène
  alkyle en $C_1$-$C_4$, hydroxyalkyle en $C_1$-$C_4$, halogenalkyle en $C_1$-$C_4$, phényle éventuellement substitué par halogène, alkyle en $C_1$-$C_4$, hydroxycarbonylalkyle en $C_2$-$C_5$, alcoxycarbonylalkyle en $C_3$-$C_8$,
- $R^6$, alkyle en $C_1$-$C_4$, halogenalkyle en $C_1$-$C_4$, alkyle en $C_2$-$C_5$-carbonyle, halogenalkyle en $C_2$-$C_5$-carbonyle ou halogène ou alkyle en $C_1$-$C_4$, ou phényle éventuellement substitué par halogène ou alkyle en $C_1$-$C_4$.
- $R^7$, $R^8$, alkyle en $C_1$-$C_4$.

2. Procédé de préparation de dérivé d'acide quinoléine-8-carboxylique I de la revendication 1, caractérisé par le fait que
   a) dans le cas où le système cyclique quinoléine et le substituant $R^3$ sont reliés par une liaison C-N, on fait réagir de manière connue, le sel de dialdéhyde nitromalonique

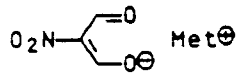

avec un hydrochlorure de 3-halogène-2-méthylaniline

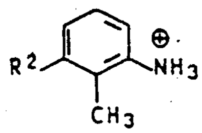

et, pour la cyclisation, on transforme, également de manière connue en soi, avec un acide ou un anhydride en le composé III

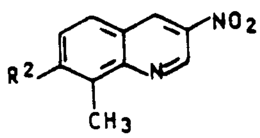
(III)

et on transforme, celui-ci de manière connue en soi, par halogénation, en le composé IV

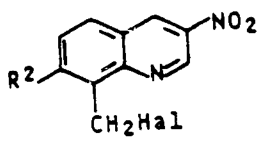
(IV)

on transforme celui-ci, également de manière connue en soi, par oxydation, en le composé (Ie)

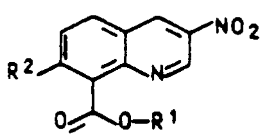
(Ie)

à cette étape, se produisent éventuellement des variations de $R^1$, le composé Ie est transformé, de manière connue, par hydrogénation catalytique en le composé amino If

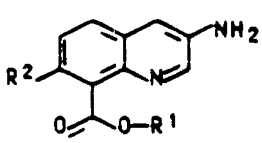
(If)

celui-ci est acylé, de manière connue, ou diazoté, également de manière connue, en le composé Ig

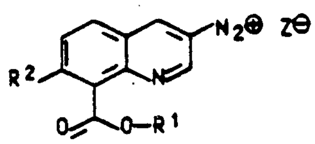

(Ig)

et celui-ci est transformé, de manière connue, en un composé Ih portant, en position 3, fluor, brome, iode ou SCN.

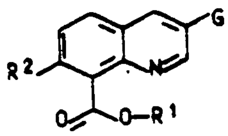

G = F, Br, J, SCN

(Ih)

**3.** 3-bromo-7-chloro-quinoléine-8-carboxylate de méthyle

**4.** Utilisation d'un dérivé d'acide quinoléine-8-carboxylique selon la revendication 1, comme herbicide.

**5.** Herbicide contenant un composé de la formule I, en quantité efficace, outre des véhicules usuels pour cela.

**6.** Herbicide selon la revendication 5, caractérisé par le fait qu'il contient 0,1 à 95 % en poids d'un dérivé d'acide quinoléine-8-carboxylique de la formule I, selon la revendication 1.

**7.** Procédé de lutte contre la croissance de plantes indésirables caractérisé par le fait qu'on traite les plantes indésirables et/ou les surfaces à maintenir exemptes d'une croissance des plantes indésirables, avec une quantité efficace, du point de vue herbicide, d'un dérivé d'acide quinoléine-8-carboxylique selon la revendication 1.